(19) Europäisches Patentamt / European Patent Office / Office européen des brevets

(11) **EP 1 817 063 B1**

(12) **EUROPÄISCHE PATENTSCHRIFT**

(45) Veröffentlichungstag und Bekanntmachung des Hinweises auf die Patenterteilung:
**25.11.2009 Patentblatt 2009/48**

(21) Anmeldenummer: **05850427.5**

(22) Anmeldetag: **02.12.2005**

(51) Int Cl.:
***A61L 2/10*** *(2006.01)*  ***A61L 2/26*** *(2006.01)*

(86) Internationale Anmeldenummer:
**PCT/EP2005/056395**

(87) Internationale Veröffentlichungsnummer:
**WO 2006/058909 (08.06.2006 Gazette 2006/23)**

(54) **VORRICHTUNG ZUR UV-BESTRAHLUNG VON FLÜSSIGKEITEN IM DURCHFLUSS**

DEVICE FOR IRRADIATING LIQUIDS WITH UV RADIATION IN A THROUGHFLOW

DISPOSITIF POUR EXPOSER EN CONTINU DES LIQUIDES ABSORBANTS A DES RAYONNEMENTS ULTRAVIOLETS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **03.12.2004 DE 102004058405**

(43) Veröffentlichungstag der Anmeldung:
**15.08.2007 Patentblatt 2007/33**

(73) Patentinhaber: **Delta UV Service Systeme**
**33818 Leopoldshöhe (DE)**

(72) Erfinder: **EGBERTS, Gerhard**
**32105 Bad Salzuflen (DE)**

(74) Vertreter: **Betten & Resch**
**Patentanwälte**
**Theatinerstrasse 8**
**(Fünf Höfe)**
**80333 München (DE)**

(56) Entgegenhaltungen:
WO-A-91/09673    WO-A-97/08099
US-A- 5 124 131    US-A- 5 505 912
US-A- 5 725 757

**Beschreibung**

Gebiet der Erfindung

Die Erfindung betrifft eine Vorrichtung zur Bestrahlung absorbierender Flüssigkeiten, beispielsweise zu desinfizierendes Abwasser mit UV-Strahlung.

Zugrundeliegender Stand der Technik

Vorrichtungen zur Behandlung des Kläranlagenablaufs zum Zwecke der Keimzahlreduzierung sind seit langer Zeit bekannt. Die grundlegenden Untersuchungen hierzu wurden bereits im Jahre 1984 von Scheible et al. in der Schriftenreihe der Environmental Protection Agency und dem Titel "Ultraviolet Disinfection of Waste Waters from secondary Effluent and Combined Sewer Overflows" veröffentlicht. Im Zuge dieser Untersuchung wurden verschiedene Konzepte der Desinfektion des Kläranlagenablaufs mittels UV-Strahlung eingehend theoretisch und praktisch untersucht und Schlussfolgerungen daraus gezogen. Ferner wurden Anforderungen für die Auslegung und den Betrieb einer UV-Anlage zur Desinfektion von Abwasser formuliert. Dabei wurden folgende Besonderheiten des Mediums Abwasser berücksichtigt:

- hohe mikrobiologische Belastung des Mediums
- schlechte UV-Durchlässigkeit des Mediums
- Neigung des Mediums zu Ablagerungen
- erhöhter Schwebstoffgehalt des Mediums
- große Durchsatzmengen bis zu mehreren 1000 m$^3$ pro Stunde.

Eine UV-Bestrahlungsanlage zur Abwasserdesinfektion ist im Prinzip eine in den Ablauf der Kläranlage montierte Bestrahlungszone, die das Wasser frei oder zwangsweise kontinuierlich durchfließt, wobei das durchfließende Wasser mittels geeigneter elektrisch betriebener UV-Strahlungsquellen mit UV-Strahlung bestimmter Wellenlänge durchleuchtet wird. Dabei kommt es darauf an, dass jedes Wasserelement eine hinreichende Bestrahlungsdosis erfährt. Die Bestrahlungsdosis ist definiert als die pro Flächeneinheit aufgebrachte mikrobiozidwirksame Strahlungsenergie. Die oben genannten Besonderheiten des Abwassers lassen sich in folgende Anforderungen an die Konstruktion von UV-Bestrahlungsanlagen umsetzen:

- gleichmäßige Ausleuchtung der Bestrahlungszone
- Vermeidung zu großer Schichtdicken des bestrahlten Wassers
- gleichmäßige Durchströmung der Bestrahlungszone
- möglichst wenig hydraulische Totzonen in der Bestrahlungszone
- Justierung der Höhe des Wasserspiegels bei schwankenden Durchsätzen
- möglichst wenig Einbauten im Wasser, die Anlagerungen von Schwebstoffen begünstigen
- leichte Zugänglichkeit für Reinigung und Lampenwechsel-

Aufgrund der in der Regel großen Durchsatzmengen kommen für die UV-Bestrahlung von biologisch gereinigtem Abwasser fast ausschließlich Bestrahlungsanlagen im Freispiegelabfluss, also im offenen Gerinne in Betracht. Dabei wird ein in der Regel bauseits gegebener offener Abflusskanal mit einer Bestrahlungsanlage bestückt, die eine den Fließquerschnitt dieses Abflusskanals gleichförmig abdeckende Anordnung von in der Regel zylindrisch geformten UV-Lichtquellen bildet. Nach der Ausrichtung der zylindrischen UV-Strahlungsquellen unterscheidet man:

- Anlagen mit senkrecht angeordneten UV-Strahlungsquellen. Hier stehen die zylindrischen UV-Quellen in einseitig geschlossenen Quarzrohren nach Art eines Grobrechens mit senkrechten Zinken im Gerinne. Der Vorteil bei dieser Bauart ist, dass ein Lampenwechsel von oben, also ohne Lösung wasserdichter Verschlüsse, möglich ist. Nachteilig ist die unvermeidliche Bildung schwer zu entfernender Ränder und Ablagerungen in der Schwankungszone des Wasserstandes.

- Anlagen mit in Fließrichtung waagrecht angeordneten UV-Strahlungsquellen. Hier sind die Quarzrohre mit darin befindlichen UV-Strahlungsquellen völlig untergetaucht und werden vom Wasser parallel umströmt. Dabei werden die Nachteile der seitlichen Anordnung vermieden, allerdings ist beim Lampenwechsel die Lösung wasserdichter Verschlüsse erforderlich.

In der derzeitigen Praxis haben sich Anlagen mit in Fließrichtung waagerecht angeordneten UV-Strahlungsquellen weitgehend durchgesetzt.

Im Gefolge der oben genannten Environmental Protection Agency-Studie gelang zuerst in den USA und Kanada die Markteinführung dieser Technik. Mittlerweile sind weltweit eine Vielzahl derartiger Anlagen installiert.

Bei Anlagen nach dem Stand der Technik wird ein bauseits vorgegebener offener rechteckiger Abflusskanal im Kläranlagenablauf mit einem Satz in Fließrichtung ausgerichteter Quecksilber-Niederdruckstrahler (Hg-ND-Strahler) bestückt. Dabei ist jeder Strahler mit einem zylindrischen Hüllrohr aus Quarz versehen, das von dem zu behandelnden Wasser umströmt wird. Je eine Reihe übereinander angeordneter Hüllrohre mit Hg-ND-Strahler sind zu einem Modul zusammengefasst, welches aus einem Edelstahlrahmen zur Halterung der übereinander angeordneten Hüllrohre, einer oberen Abdeckung zur Vermeidung des Austritts gesundheitsschädlicher UV-Strahlung, einem Steckverbinder für die Stromversorgung sowie Handgriffen zur Entnahme in vertikaler Richtung für Wartungszwecke besteht.

Zur vollständigen Ausleuchtung des Kanalquerschnitts sind mehrere derartige Module parallel zueinander in einem fest am Kanal montierten Rahmen befestigt, der als Auflage, Arretierung und Kabelkanal fungiert.

Hg-ND-Strahler sind Quecksilber-Gasentladungslampen mit UV-durchlässigem Lampenrohr, die den überwiegenden Teil ihrer Strahlung in der mikrobiozid wirkenden Triplet-Resonanz des Quecksilbers bei 253,7 nm emittieren. Dieser Lampentyp hat typischerweise eine elektrische Leistung von weniger als 100 Watt, auch in Sonderbauformen (Amalgamlampe). Für größere Anlagen mit einer Durchflussmenge von 10.000 m$^3$ pro Stunde und mehr kann als Richtwert je nach Wasserbeschaffenheit ein elektrischer Energieverbrauch von 20 bis 30 Wattstunden pro Kubikmeter Wasser angesetzt werden. Bei 100 Watt Leistung eines Hg-ND-Strahlers sind bei einer solchen Anlage 2000 bis 3000 Strahler erforderlich. Das ist nach dem Stand der Technik durch Nebeneinander- oder Nacheinanderschalten von Strahlerbaugruppen ohne Weiteres möglich, jedoch bestehen die folgenden Nachteile:

- keine Größendegression der Anlagekosten
- großer Platzbedarf
- hoher Wartungs- und Überwachungsaufwand
- hohe Lampenkosten.

Aus diesen Gründen ist die UV-Behandlung größerer Abwasserströme nach dem Stand der Technik sehr kostenaufwendig. Der logische Schritt zur Kostensenkung ist die Erhöhung der Leistung pro Strahler, die unmittelbar zur Senkung der Strahleranzahl und damit der Anlagekosten führt.

Bei Hg-ND-Strahlern mit Amalgam können Leistungen bis maximal ca. 300 Watt erzielt werden. Für eine weitergehende Leistungssteigerung sind Quecksilber-Mitteldruckstrahler (Hg-MD-Strahler) bekannt, die je nach Bauform eine elektrische Leistung von 10 kW und mehr pro Strahler besitzen können. Sie emittieren in hoher Leistungsdichte ein Quasikontinuum von UV-Strahlung, wobei in Bezug auf die mikrobiozid wirkende UV-Strahlung ein Erzeugungswirkungsgrad von ca. 12% bis 15% besteht. Bisherige Versuche, derartige hochleistungsfähige Hg-MD-Strahler für die Abwasserdesinfektion einzusetzen, waren aus den folgenden Gründen nicht erfolgreich:

- eine ungleichmäßige Verteilung der Bestrahlungsintensität durch die hohe Strahlungsleistung der einzelnen Strahler
- verstärkte Ablagerungen aus dem Abwasser und Einbrennen dieser Ablagerunen auf den Hüllrohren der Strahler. Grund dafür ist der wesentlich höhere Temperaturgradient Abwasser-Hüllrohr zur Ableitung der höheren Wärmeleistung des Hg-MD-Strahlers.
- Unfall- und Kurzschlussgefahren durch Handhabung der Hochspannungsanschlüsse der Hg-MD-Strahler im wasserbenetzten Bereich.

Es besteht daher ein Bedarf an kostengünstigen Anlagen zur UV-Behandlung größerer Abwasserströme.

Die US 5,124,131 beschreibt eine Vorrichtung zur UV-Bestrahlung von Abwasser im Durchfluss mit wenigstens zwei Strahlerbaugruppen mit jeweils einer zylindrischen UV-Lampe, um die ein konzentrisches Hüllrohr angeordnet ist, wobei zwischen der Lampe und dem Hüllrohr ein Kühlmittel zur Ableitung der Wärme der Lampe fließt.

Die US 5,505,912 beschreibt ein Kühlsystem für einen Reaktor mit UV-Lampen, deren Strahlung ein zu behandelndes Medium bestrahlt. Das Reaktorsystem ist ausgebildet, Schmutzstoffe in dem Medium zu behandeln, wobei die UV-Lampen bei Betriebstemperaturen oberhalb von 300°C arbeiten.

Zusammenfassung der Erfindung

Der Erfindung liegt die Aufgabe zugrunde, eine Anlage zur UV-Bestrahlung absorbierender Flüssigkeiten im Durchfluss vorzuschlagen, welche eine gleichmäßige Bestrahlung der Flüssigkeit auch bei hohen Durchflussmengen ermöglicht.

Eine weitere Aufgabe der Erfindung liegt darin, eine derartige Vorrichtung vorzuschlagen, die kostengünstig herzustellen ist.

Noch eine weitere Aufgabe der Erfindung liegt darin, eine Vorrichtung zur UV-Bestrahlung absorbierender Flüssigkeiten im Durchfluss vorzuschlagen, bei welcher der Wartungsaufwand verringert ist.

Gelöst wird die Aufgabe durch eine Vorrichtung zur UV-Bestrahlung von absorbierenden Flüssigkeiten, beispielsweise zu desinfizierendem Abwasser, im Durchfluss durch wenigstens zwei UV-Strahlerbaugruppen, **dadurch gekennzeichnet, dass** jede Strahlerbaugruppe eine zylindrische UV-Lichtquelle und drei konzentrische, zylindrische Hüllrohre aufweist, wobei in einem Hohlraum zwischen einem inneren und einem mittleren Hüllrohr ein Kühlmittel zur Ableitung von Wärme der UV-Strahlungsquelle fließt.

Durch das Kühlmittel kann die Temperatur am äußeren Hüllrohr verringert werden, so dass ein Einbrennen von Ablagerungen auf dem Hüllrohr verhindert werden kann. Es ist möglich, den Durchmesser des äußersten Hüllrohres zu vergrößern, wodurch eine gleichmäßigere Verteilung der Bestrahlungsintensität erreichbar wird. Der Durchmesser des äußeren Hüllrohres liegt dabei vorzugsweise zwischen 80 und 250 mm, insbesondere zwischen 120 und 200 mm.

Im Hohlraum zwischen dem äußeren und dem mittleren Hüllrohr kann eine Gasfüllung aus UV-durchlässigem Gas vorgesehen sein.

Die Leistungsdichte der UV-Strahlungsquellen, beispielsweise Hg-MD-Strahler, beträgt vorzugsweise wenigstens 10 Watt UV-C-Strahlung pro cm in Axialrichtung.

Die UV-Strahlungsquellen können dabei quer zur Flussrichtung horizontal angeordnet sein, beispielsweise an einer U-förmigen Stahlumwandung, wobei die Hüllrohrenden vorzugsweise abgedichtet gelagert und mit Anschlüssen für Energie und Kühlmittel versehen sind. Zur Vereinfachung der Wartung können seitlich Montagegruben vorgesehen sein, die den Zugriff auf und den Austausch der UV-Strahlungsquellen erlauben.

Um den Austritt gesundheitsschädlicher UV-Strahlung nach oben zu verhindern, sind vorzugsweise Abdeckungen oberhalb der Strahlerbaugruppen vorgesehen.

Die Strahlerbaugruppen können im Längsschnitt im Muster einer zweidimensional dichtesten Kugelpackung angeordnet sein, um eine möglichst gleichmäßige Verteilung der Bestrahlungsintensität zu gewährleisten.

Für das Kühlmittel kann eine Kühlmittel-Umwälzpumpe und ein Wärmetauscher zur Abgabe der Wärme an die absorbierende Flüssigkeit vorgesehen sein.

Als Kühlmittel kann vorzugsweise destilliertes Wasser verwendet werden, welches eine sehr geringe UV-Absorption aufweist. Dabei kann Alkohol wie etwa Ethanol oder Methanol zugegeben werden, um biologische Verunreinigung des Kühlmittels zu verhindern.

Die Hüllrohre bestehen vorzugsweise aus UV-durchlässigem Material wie beispielsweise Quarzglas.

Es ist möglich, eine durchflussabhängige Leistungsregelung der UV-Strahlungsquellen vorzusehen, so dass bei wechselnder Durchflussmenge immer die richtige Strahlungsintensität vorhanden ist.

Kurze Beschreibung der Zeichnungen

Weitere Merkmale und Vorteile der Erfindung werden anhand der folgenden Beschreibung eines bevorzugten Ausführungsbeispiels unter Bezugnahme auf die beiliegenden Zeichnungen deutlich.

Figur 1     zeigt in Aufsicht ein Ausführungsbeispiel einer er- findungsgemäßen Vorrichtung zur UV-Strahlung absorbierender Flüssigkeiten im Durchfluss.

Figur 2     zeigt eine Längsschnittansicht des Ausführungsbei- spiels von Figur 1.

Figur 3     zeigt eine Querschnittsansicht entlang der Linie A-A von Figur 2.

Figur 4     zeigt ein Ausführungsbeispiel einer erfindungsgemä- ßen Strahlerbaugruppe.

Figur 5     zeigt eine Detailansicht der Strahlerbaugruppe von Figur 4.

Figur 6     zeigt ein Beispiel der Anordnung mehrerer Strahler- baugruppen zur Erzielung einer gleichmäßigen Bestrahlungsintensität.

Detaillierte Beschreibung der Erfindung

Die Erfindung wird im Folgenden anhand eines bevorzugten Ausführungsbeispiels im Detail beschrieben.

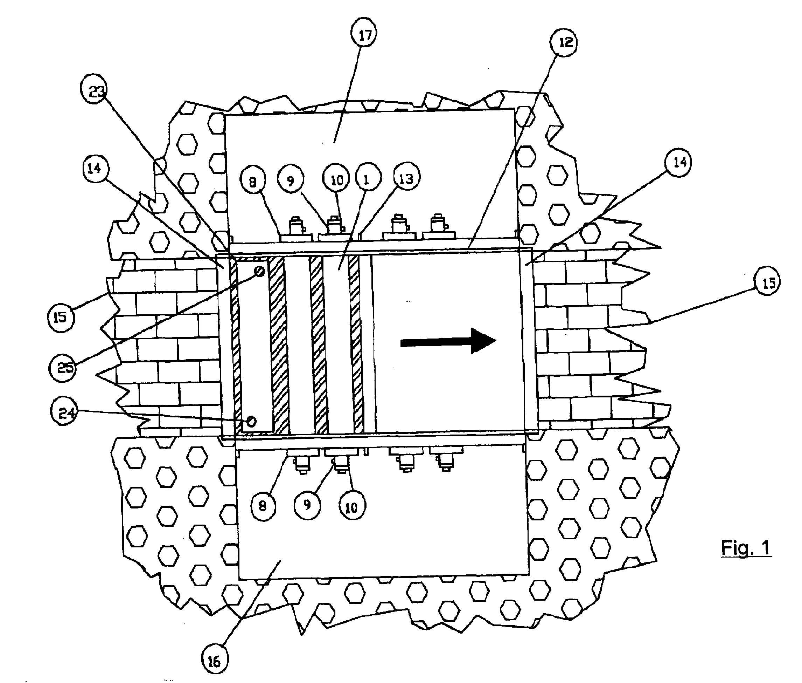

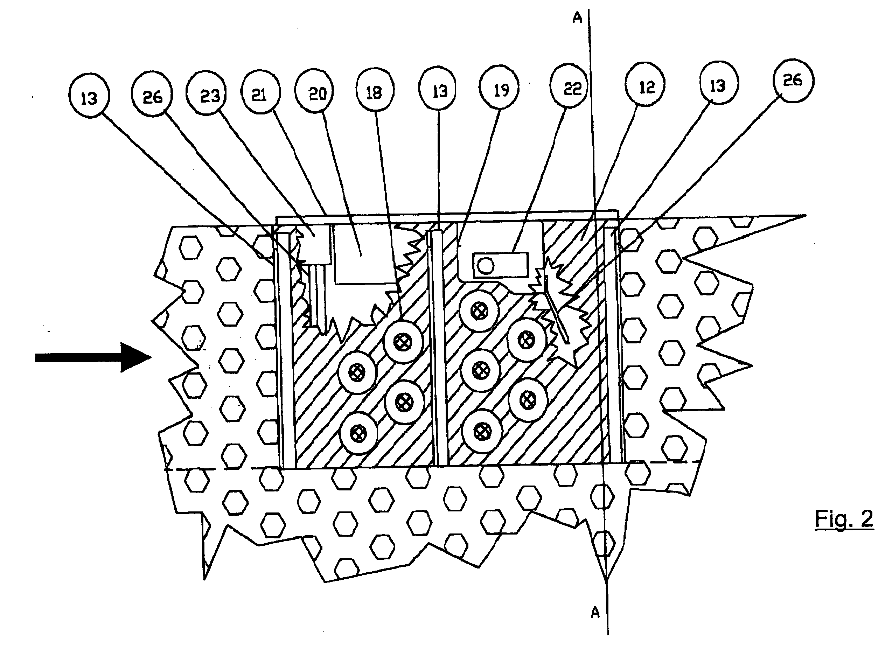

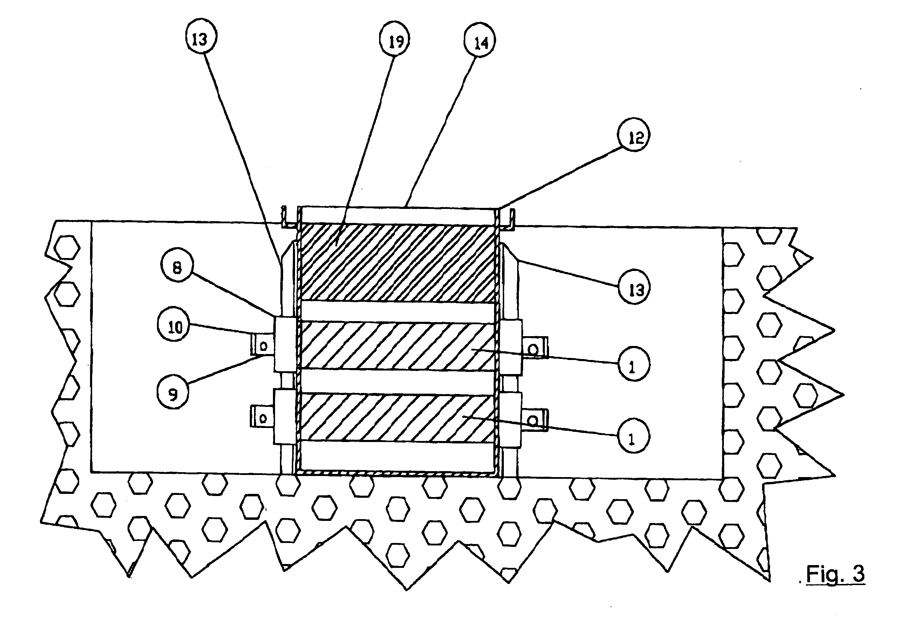

Die erfindungsgemäße Vorrichtung ist in Draufsicht in Figur 1, in Längsschnittansicht in Figur 2 und in Querschnittsansicht entgegen der Flussrichtung in Figur 3 dargestellt. Die Flussrichtung der absorbierenden Flüssigkeit ist dabei durch einen Pfeil angedeutet, spielt für die Funktionsfähigkeit der erfindungsgemäßen Vorrichtung jedoch keine Rolle. Eine U-förmig abgewinkelte Edelstahlwandung 12, die durch äußere Versteifungen 13 und oberhalb der Flüssigkeitsoberfläche angeordnete Verstrebungen 14 formstabil gehalten ist, wird in das Gerinne 15 eingebaut. Auf jeder Seite befindet sich eine abdeckbare Grube 16, 17, über die alle elektrischen Verbindungen, Steuerungsleitungen sowie Rohre bzw. Schläuche des Kühlkreislaufs für Wartungszwecke zugänglich geführt werden. In den zwei seitlichen vertikalen Bereichen der Edelstahlwandung 12 befinden sich paarweise gegenüberliegende kreisförmige Durchbrüche 18, in die die Strahlerbaugruppen, nämlich äußeres Hüllrohr 1, Halterungen und Abdeckungen 8, 9 und 10 wasserdicht eingedichtet sind. Die elektrischen Anschlusskabel und Kühlmittelschläuche sind nicht eingezeichnet. Sie sind am vorteilhaftesten in einem gleichfalls nicht eingezeichneten Kabelkanal an der vertikalen Versteifung 13 geführt. Nach oben abgedeckt wird die Bestrahlungszone über einen oder mehrere Senkkästen aus Edelstahl 19, 20, die sicherstellen, dass auch bei starkem Wasserdurchsatz oberhalb der obersten Strahlerbaugruppe der von der Bestrahlungsgeometrie vorgegebene Maximalabstand von der Strahlerbaugruppe (das ist die lichte Weite zwischen Senkkasten und oberer Strahlerbaugruppe) nicht überschritten wird. Umgekehrt sorgt ein Wehr in Fließrichtung nach der Bestrahlungszone dafür, dass die Unterkante des Senkkastens immer benetzt bleibt und insbesondere die oberste Strahlerbaugruppe unter keinen Umständen ganz oder teilweise trocken fällt. Der Senkkasten kann entweder fest als stabilisierende Abdeckung in die Edelstahlwandung eingeschweißt sein oder entnehmbar zur besseren Zugänglichkeit der Bestrahlungszone zu Reinigungszwecken ausgebildet sein. Darüber befindet sich eine begehbare Abdeckung 21, die auch als Fundamentplatte für einen Schaltschrank mit der Stromversorgung und den erforderlichen Steuerungs- und Überwachungseinrichtungen dienen kann, sofern dieser unmittelbar an der Anlage platziert werden soll. Der fest eingeschweißte Senkkasten 19 dient der Aufnahme einer Kühlmittel-Umwälzpumpe 22 und als Kabelschacht, falls der Schaltschrank auf der Abdeckung montiert ist.

In Flussrichtung vor der Bestrahlungszone befindet sich ein Wärmetauscher 23, über den die vom Kühlmittel aufgenommene Wärmeenergie der Hg-MD-Strahler an den Flüssigkeitsstrom abgegeben wird. Er besteht aus einer Verteilkammer mit Anschluss für die Zuleitung 24, Sammelkammer mit Anschluss für die Ableitung 25 sowie dazwischengeschalteten U-förmigen Kupferrohren 26, die von oben in das Wasser eintauchen und die Abwärme an den zu behandelnden Abwasserstrom abgeben. Der Wärmetauscher dient gleichzeitig als Sichtblende gegen nach vorne aus der Bestrahlungszone austretende UV-Strahlung und als Strömungshindernis zur Vergleichmäßigung der Durchströmung der Bestrahlungszone.

In Fließrichtung hinter der Bestrahlungszone befindet sich schließlich ein schräg nach unten weisendes Leitblech 26, das einerseits die Strömungsführung am oberen Rand der Bestrahlungszone optimiert und andererseits als Sichtblende gegen nach hinten aus der Strahlungszone austretende UV-Strahlung dient.

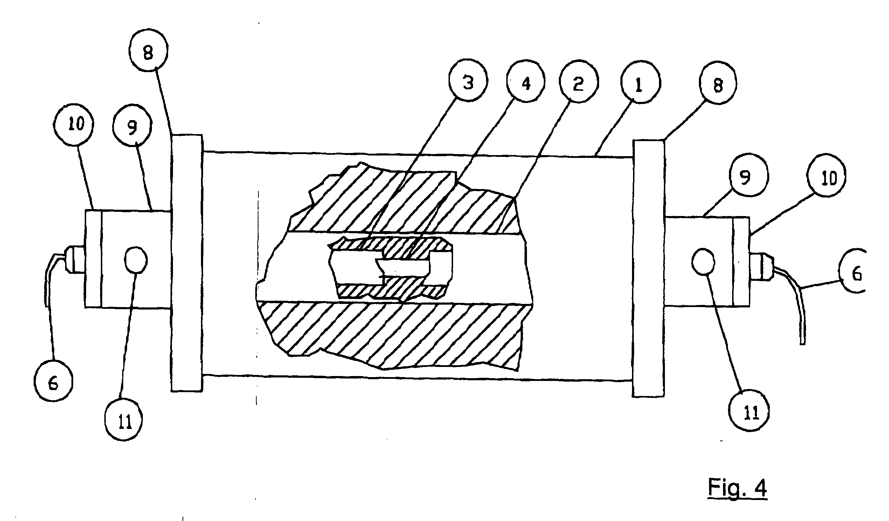

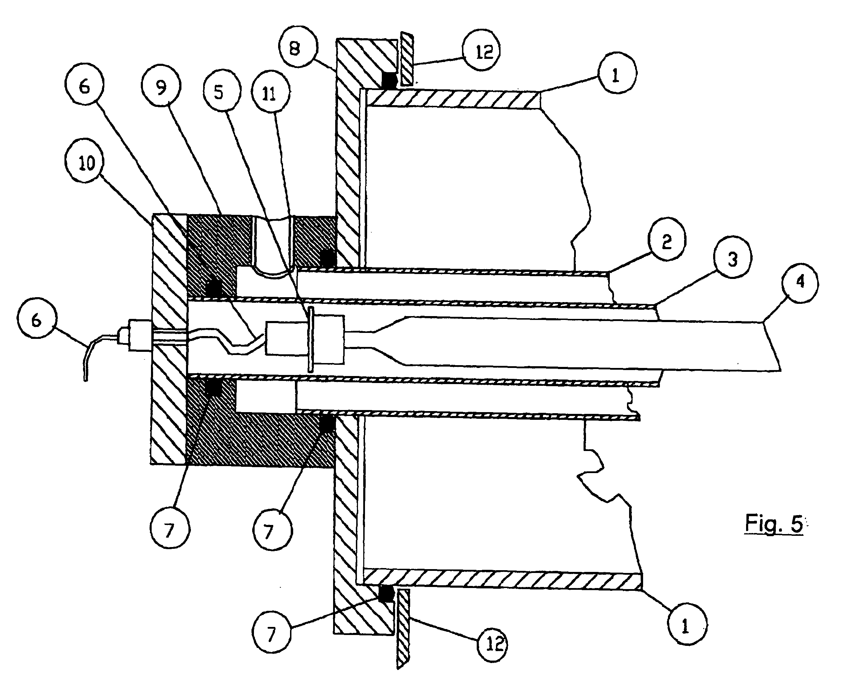

Die Strahlerbaugruppe wird im Folgenden im Detail unter Bezugnahme auf die Figuren 4 und 5 erläutert. Diese besteht aus einem zentrisch angeordneten, zylindrischen UV-Strahler, beispielsweise einem Hg-MD-Strahler mit einer Leistung von wenigstens 10 Watt UV-C-Strahlung pro Zentimeter in Axialrichtung, welche Strahlungsquelle 4 mit Halterung 5 und elektrischen Anschlüssen 6 versehen ist. Äußeres Hüllrohr 1, mittleres Hüllrohr 2 und inneres Hüllrohr 3, vorzugsweise ausgebildet aus UV-durchlässigem Quarzglas, sind konzentrisch zu der UV-Strahlquelle 4 angeordnet und mit seitlichen, mit O-Ringen 7 eingedichteten Halterungen 8, 9 und einer Endabdeckung 10 abgedichtet. Weiterhin sind Anschlüsse 11 für die Kühlmittelzirkulation des Kühlmittels in dem Hohlraum zwischen dem inneren Hüllrohr 3 und dem mittleren Hüllrohr 2 vorgesehen.

Die erfindungsgemäß mögliche gleichmäßigere Verteilung der Bestrahlungsintensität auf die absorbierende Flüssigkeit wird im Folgenden anhand eines Beispiels mit einem Wasserdurchsatz von 10.000 m$^3$/h entsprechend 2777 1/sec illustriert.

Angenommen sei der Einsatz von 50 Hg-MD-Strahlern zu je 10 kW elektrischer Leistung, ein Erzeugungswirkungsgrad der UV-Strahlung von 15%, eine Länge der Lichtquelle von 100 cm, ein Außendurchmesser des (äußeren) Hüllrohres von 4 cm gemäß dem Stand der Technik und eine UV-Durchlässigkeit des Abwassers von 80%/cm.

Dann errechnet sich für die Außenoberfläche des Hüllrohres eine Bestrahlungsstärke von ca. 1,2 W/cm$^2$, im Abstand von 1 cm eine Bestrahlungsstärke von ca. 0,65 W/cm$^2$ und im Abstand von 2 cm eine Bestrahlungsstärke von ca. 0,38 W/cm$^2$, entsprechend weniger als einem Drittel.

Betrachtet man den Raum, in dem die Bestrahlungsstärke von 100% auf ca. 33% absinkt, beträgt dieser pro Hüllrohr ca. 3,8 1. Bei 50 Strahlern ergibt sich ein Gesamtraum von 190 1. Die 2777 1/sec Wasserdurchsatz gemäß diesem Beispiel können nicht durch eine derartig kleine Bestrahlungszone geführt werden. Die sehr hohe Flächenbelastung mit abzuführender thermischer Energie führt außerdem zu einer verstärkten Bildung und einem Einbrennen von Ablagerungen auf dem äußeren Hüllrohr, was wiederum zu einer zunehmenden Strahlungsabsorption in der Schmutzschicht führt.

Bei einem Außendurchmesser des äußeren Hüllrohres 1 von 10 cm (20 cm) beträgt die Bestrahlungsdichte bei dem obigen Beispiel am Hüllrohr 0,477 (0,238) W/cm$^2$ und geht bei einem radialen Abstand von 3 cm (3,5 cm) auf ca. ein Drittel zurück, nämlich 0,153 W/cm$^2$ (0,08 W/cm$^2$). Das zugehörige Volumen beträgt pro UV-Quelle ca. 12,2 l (26 l) entsprechend 610 l (1300 l) Gesamtvolumen für 50 Strahler. Die Vergrößerung des äußeren Hüllrohres ermöglicht so deutlich größere Durchflussleistungen der zu bestrahlenden Flüssigkeit. Dazu ist es jedoch erforderlich, die von der leistungsstarken UV-Strahlungsquelle erzeugte Wärme mittels des Kühlmittels wirksam abzuführen. Andererseits muss das Kühlmittel selbst möglichst wenig UV-Strahlung aufnehmen, so dass ein relativ "dünner" Hohlraum zwischen innerem Hüllrohr 3 und mittlerem Hüllrohr 2 von dem Kühlmittel durchströmt wird, bei dem es sich wegen der geringen UV-Absorption vorzugsweise um destilliertes Wasser, gegebenenfalls mit Ethanol-Zusatz zur Verhinderung von Bakterien- oder Algenbildung handelt. Wegen der geringen UV-Absorption sind die Hüllrohre 1, 2, 3 vorzugsweise aus Quarzglas ausgebildet.

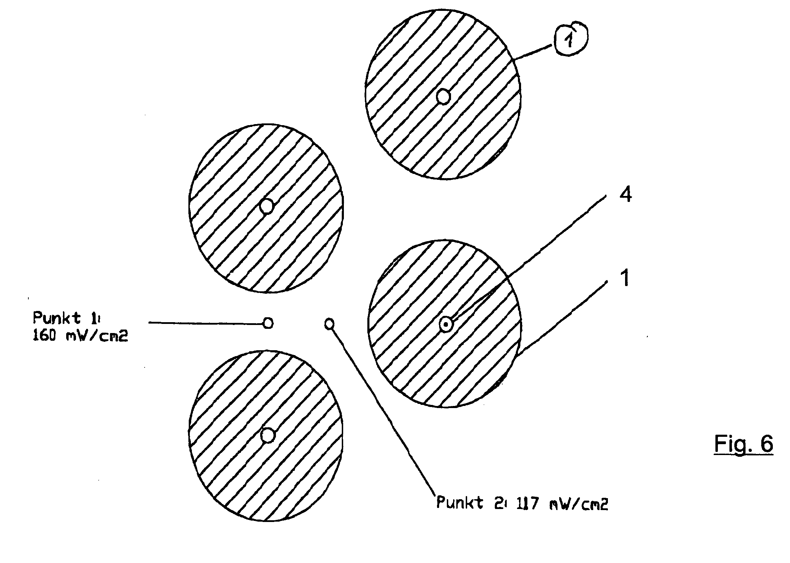

Durch das Zusammenwirken mehrerer Strahlerbaugruppen kann eine weitgehende Vergleichmäßigung der Bestrahlungsintensität erreicht werden, die schematisch in Figur 6 gezeigt ist. Dort ist eine Anzahl von Hg-MD-Strahlern mit einem Durchmesser des äußeren Hüllrohres 1 von 20 cm dargestellt, die eine Strahlungsleistung von 15 W/cm und einen minimalen Abstand von 7 cm zweier benachbarter Strahler entsprechend dem Muster einer zweidimensional dichtesten Kugelpackung aufweisen. Ausgehend von einer Bestrahlungsstärke von 0,238 W/cm$^2$ (siehe oben) an der Oberfläche des äußeren Hüllrohres 1 ergibt sich bei einer UV-Durchlässigkeit des Wassers von 80% pro Zentimeter rechnerisch eine Bestrahlungsstärke von maximal 0,16 W/cm$^2$ durch Überlagerung von zwei Strahlern im Punkt 1 und minimal von 0,117 W/cm$^2$ durch Überlagerung von drei Strahlern im Punkt 2.

Die wünschenswerte Vergrößerung des äußeren Hüllrohres 1 erfordert zusätzliche Maßnahmen zur Beseitigung der mit dem Betrieb der leistungsfähigen UV-Quelle verbundenen Wärmeenergie. Da die Wärmeabfuhr durch Konvektion mit nachgeschaltetem Wärmeübergang in das zu bestrahlende Medium bei den bei einem größeren Hüllrohr vergrößerten Abständen von mehreren Zentimetern nicht mehr ausreichend ist, sind erfindungsgemäß insgesamt drei Quarzrohre 1, 2, 3 für jede Strahlerbaugruppe vorgesehen, nämlich das innere Hüllrohr 3, das mittlere Hüllrohr 2 und das von der zu bestrahlenden Flüssigkeit umströmte äußere Hüllrohr 1. Dadurch ergibt sich die Möglichkeit, durch den Hohlraum zwischen innerem Hüllrohr 3 und mittlerem Hüllrohr 2 voll entsalztes Wasser oder ein anderes hinreichend UVdurchlässiges flüssiges oder gasförmiges Kühlmedium zirkulieren zu lassen. Aufgrund der Absorption und der hohen Wärmekapazität des Kühlmediums wird, anders als bei Kühlung durch ein Gebläse, auch die Energie der Wärmestrahlung weitgehend abgeführt und insgesamt eine sehr gleichmäßige Temperatur der UV-Quelle 4 erzielt. Eine die Belagsbildung fördernde Aufheizung des flüssigkeitsbenetzten äußeren Hüllrohres 1 kann dadurch fast vollständig vermieden werden.

Ein wesentlicher Vorteil der erfindungsgemäßen Vorrichtung liegt in der wirksamen Vermeidung von Ablagerungen auf dem von der zu bestrahlenden Flüssigkeit umströmten äußeren Hüllrohr. Bei herkömmlichen Systemen wird die gesamte in der UV-Quelle erzeugte Wärmeenergie über das Hüllrohr an die zu bestrahlende Flüssigkeit abgegeben. Dies führt notwendigerweise zu einem Temperaturgradienten Hüllrohr-Wasser. Dadurch können sich Partikel auf der äußeren Oberfläche des Hüllrohrs ablagern, insbesondere bei geringen Fließgeschwindigkeiten, wodurch die Absorption der UV-, VIS- und IR-Strahlung diesen Temperaturgradienten weiter erhöhen. Dadurch wird die Bildung von Ablagerungen weiter beschleunigt. Schließlich können sich richtige dunkle und feste Krusten bilden, die nur schwer zu entfernen sind.

Für den Wärmeübergang an einer Phasengrenze fest-fluid gilt das Folgende:

$$Q = \alpha \bullet A (\vartheta - T),$$

wobei

Q   der Wärmestrom in Watt

$\alpha$   der Wärmeübergangskoeffizient in Watt pro m$^2$ und °K,

A   die Fläche des Wärmedurchtritts in m$^2$,

$\vartheta$   die Temperatur im fluid in °K, und

T   die Wandtemperatur in °K ist.

Für ein mit einer Geschwindigkeit v (in m/sec) von Wasser umströmtes Rohr wird in der Literatur für $\alpha$ folgende Näherung angegeben:

$$\alpha = (500 \text{ bis } 1.800) \text{ x } \sqrt{v},$$ je nach Oberflächenbeschaffenheit.

Aufgabenstellung einer wirksamen Sauberhaltung des äußeren Hüllrohres ist es, für eine gegebene Leistung die in der obigen Gleichung angegebene Temperaturdifferenz (9 - T) unter allen Betriebsbedingungen so klein zu halten, dass der oben beschriebene sich selbst verstärkende Verschmutzungsprozess vermieden werden kann. Untersuchungen haben gezeigt, dass bei der erfindungsgemäßen Anordnung eine weitgehende Verschmutzungsfreiheit erzielt wird, wenn die abzuführende Wärmeleistung bei einer mittleren Umströmungsgeschwindigkeit von 2 m/sec unter ca. 2000 W/m$^2$ gehalten wird, entsprechend einer Temperaturdifferenz ($\vartheta$ - T) von 1 bis 3 Kelvin bei der oben angegebenen Bandbreite von α.

Zur weitgehenden Freihaltung der Oberfläche von Ablagerungen tragen dabei einerseits die aus dem Zusammenwirken der reduzierten Wärmeleistung pro Fläche und der Wärmeabfuhr durch die erhöhte Tangentialgeschwindigkeit begrenzte Temperatur des äußeren Hüllrohres, andererseits die Sauberhaltung der Oberfläche durch die abrasiven Kräfte des umströmenden Wassers bei.

Reduziert man bei niedrigen Fließgeschwindigkeiten auch die Lampenleistung, so bilden die Ablagerungen nur einen dünnen Film auf der Oberfläche des Hüllrohres, der bei voller Durchströmung wieder abgespült wird.

Die Anforderungen an die Wärmelast pro Flächeneinheit und die tangentiale Geschwindigkeit lässt sich anhand eines Beispiels für Hg-MD-Strahler zur Abwasserdesinfektion auf Grundlage folgender beispielhafter charakteristischer Daten in konkrete geometrische Anforderungen umsetzen:

| | |
|---|---|
| Länge der Entladungsstrecke: | 100 cm |
| UV-C-Strahlungsleistung | > 1200 W |
| Eindringtiefe im Wasser | 3 cm (Rückgang auf I/e) |
| geforderte Durchschnittsdosis: | 40 mJ/cm$^2$ gemäß nachstehender Formel (1) |

Anzeige Lampenreihen hintereinander: mindestens 2 zur ausreichenden Durchmischung.

Dann ergibt sich aus der Begrenzung der Flächenleistung auf weniger als 2000 W/m$^2$ unter Berücksichtigung der Wärmeabfuhr durch das Kühlmittel von ca. 50% der Gesamtleistung ein minimaler Durchmesser des äußeren Hüllrohres 1 von 8 cm. Aus der geforderten Tangentialgeschwindigkeit lässt sich eine Begrenzung des lichten Abstandes benachbarter Quarzrohre ableiten. Dabei wird folgende Näherungsformel für die UV-Dosis verwendet:

$$\text{Dosis} \ (\text{mJ/cm}^2) \ = \ \frac{UV - Leistung(W) \bullet Eindringtiefe(cm)}{Durchfluss(l/\sec)} \qquad (1)$$

Aufgelöst nach dem Durchfluss ergibt sich unter Verwendung einer Minimalgeschwindigkeit des Wasser von 2 m/sec ein maximaler freier Querschnitt von 900 cm$^2$ pro Strahler entsprechend einer maximalen lichten Weite zwischen zwei benachbarten Hüllrohren von 9 cm.

**Patentansprüche**

1. Vorrichtung zur UV-Bestrahlung von absorbierender Flüssigkeit im Durchfluss aufweisend wenigstens zwei UV-Strahlerbaugruppen mit jeweils einer zylindrischen UV-Strahlungsquelle (4), um die drei konzentrische Hüllrohre (1, 2, 3) angeordnet sind, wobei zwischen einem inneren (3) und einem mittleren (2) der Hüllrohre ein Kühlmittel zur Ableitung der Wärme der UV-Strahlungsquelle fließt, wobei die zu bestrahlende Flüssigkeit das äußere (1) der drei Hüllrohre umfließt und nur mit diesem in Berührung kommt.

2. Vorrichtung nach Anspruch 1, wobei in dem Hohlraum zwischen dem äußeren Hüllrohr (1) und dem mittleren Hüllrohr (2) eine Gasfüllung vorgesehen ist.

3. Vorrichtung nach Anspruch 1 oder 2, wobei der Außendurchmesser des äußeren Hüllrohres (1) zwischen 80 und 250 mm beträgt.

4. Vorrichtung nach einem der Ansprüche 1 bis 3, wobei die UV-Strahlungsquelle (4) eine Leistungsdichte von wenigstens 10 Watt UV-C-Strahlung pro cm in Axialrichtung aufweist.

5. Vorrichtung nach einem der Ansprüche 1 bis 4, wobei die UV-Strahlungsquelle ein Hg-Mitteldruckstrahler ist.

6. Vorrichtung nach einem der Ansprüche 1 bis 5, wobei die wenigstens zwei zylindrischen UV-Strahlungsquellen quer zur Flussrichtung der absorbierenden Flüssigkeit horizontal angeordnet sind.

7. Vorrichtung nach Anspruch 6, wobei die wenigstens zwei UV-Strahlungsquellen (4) beidseitig an einer mit kreisförmigen Durchbrüchen (18) versehenen U-förmigen Stahlwandung (12) angebracht sind.

8. Vorrichtung nach Anspruch 7, wobei beide Enden der drei konzentrischen Hüllrohre (1, 2, 3) gegenüber der U-förmigen Stahlwandung (12) abgedichtet gelagert sind, an welchen Enden sich Anschlüsse für elektrische Energie und das Kühlmittel befinden.

9. Vorrichtung nach einem der Ansprüche 1 bis 8, aufweisend Abdeckungen (8, 9, 10), die einen Austritt von UV-Strahlung nach oben verhindern.

10. Vorrichtung nach Anspruch 8, aufweisend wenigstens eine in Flussrichtung der absorbierenden Flüssigkeit seitlich angeordnete Montagegrube (16, 17), welche Wartung und Austausch der UV-Strahlungsquellen (4) ohne Demontage Flüssigkeits berührter Teile ermöglicht.

11. Vorrichtung nach Anspruch 6, wobei mehrere UV-Strahlungsquellen im Längsschnitt entsprechend dem Muster einer zweidimensionalen dichtesten Kugelpackung angeordnet sind.

12. Vorrichtung nach einem der Ansprüche 1 bis 11, aufweisend eine Kühlmittel-Umwälzpumpe (22) sowie einen Wärmetauscher (23) zur Abgabe der von dem Kühlmittel aufgenommenen Wärmeenergie an den Strom der zu absorbierenden Flüssigkeit.

13. Vorrichtung nach Anspruch 12, wobei der Wärmetauscher (23) gleichzeitig als Abschirmung gegen in Flussrichtung nach vorne austretende UV-Strahlung und als Strömungshindernis zur Vergleichmäßigung der Durchströmung der Bestrahlungszone dient.

14. Vorrichtung nach einem der Ansprüche 1 bis 13, wobei destilliertes Wasser mit Zusatz von Alkohol als Kühlmittel eingesetzt wird.

15. Vorrichtung nach einem der Ansprüche 1 bis 14, aufweisend eine von der Durchflußmenge abhängige Regelung der Leistung der UV-Strahlungsquellen (4).

16. Vorrichtung nach einem der Ansprüche 1 bis 15, wobei die lichte Weite zwischen den äußeren Hüllrohren (1) zweier übereinander angeordneter UV-Strahlungsquellen (4) so gewählt ist, dass die Tangentialgeschwindigkeit der Flüssigkeit an der Oberfläche des äußeren Hüllrohres ausgedrückt in m/sec größer ist als der Zahlwert der Leistungsdichte der durch das äußere Hüllrohr (1) austretenden UV-Strahlung ausgedrückt in W/cm$^2$.

17. Vorrichtung nach einem der Ansprüche 1 bis 16, wobei die absorbierende Flüssigkeit zu desinfizierendes Abwasser ist.

18. Abwasserreinigungsanlage, aufweisend eine Bestrahlungsvorrichtung nach einem der Ansprüche 1 bis 17.

**Claims**

1. Device for UV irradiation of absorbing liquid in continuous flow, exhibiting at least two UV radiator assemblies each having a cylindrical UV-radiation source (4), around which three concentric casing tubes are arranged, wherein a coolant for dissipating the heat of the UV-radiation source flows between an inner casing tube (3) and a middle casing tube (2), wherein the liquid to be irradiated flows around the outer casing tube (1) of the three casing tubes and only comes into contact with said casing tube.

2. Device according to Claim 1, wherein a gas filling is provided in the hollow space between the outer casing tube (1) and the middle casing tube (2).

3. Device according to Claim 1 or 2, wherein the outside diameter of the outer casing tube (1) amounts to between 80 mm and 250 mm.

4. Device according to one of Claims 1 to 3, wherein the UV-radiation source (4) exhibits a power density of at least 10 W UV-C radiation per cm in the axial direction.

5. Device according to one of Claims 1 to 4, wherein the UV-radiation source is a medium-pressure Hg radiator.

8

6. Device according to one of Claims 1 to 5, wherein the at least two cylindrical UV-radiation sources are arranged horizontally at right angles to the direction of flow of the absorbing liquid.

7. Device according to Claim 6, wherein the at least two UV-radiation sources (4) are fitted on both sides to a U-shaped steel wall (12) provided with circular apertures (18).

8. Device according to Claim 7, wherein both ends of the three concentric casing tubes (1, 2, 3) are supported in sealed manner in relation to the U-shaped steel wall (12), on which ends there are located connectors for electrical power and for the coolant.

9. Device according to one of Claims 1 to 8, exhibiting covers that prevent an emergence of UV radiation upwards.

10. Device according to Claim 8, exhibiting at least one mounting recess (16, 17) arranged laterally in the direction of flow of the absorbing liquid, said mounting recess enabling maintenance and exchange of the UV-radiation sources (4) without disassembly of parts in contact with liquid.

11. Device according to Claim 6, wherein several UV-radiation sources in longitudinal section are arranged in a manner corresponding to the pattern of two-dimensional closest sphere packing.

12. Device according to one of Claims 1 to 11, exhibiting a coolant circulating pump (22) and also a heat-exchanger (23) for releasing the thermal energy taken up by the coolant to the stream of the liquid to be absorbed.

13. Device according to Claim 12, wherein the heat-exchanger (23) serves simultaneously as shielding against UV radiation emerging forwards in the direction of flow and as an obstruction to flow for the purpose of smoothing out the flow through the irradiation zone.

14. Device according to one of Claims 1 to 13, wherein distilled water with addition of alcohol is employed as coolant.

15. Device according to one of Claims 1 to 14, exhibiting a flow-rate-dependent regulation of the power of the UV-radiation sources (4).

16. Device according to one of Claims 1 to 15, wherein the clear width between the outer casing tubes (1) of two UV-radiation sources (4) arranged one above the other is chosen in such a way that the tangential speed of the liquid along the surface of the outer casing tube, expressed in m/s, is greater than the numerical value of the power density of the UV radiation emerging through the outer casing tube (1), expressed in $W/cm^2$.

17. Device according to one of Claims 1 to 16, wherein the absorbing liquid is waste water to be disinfected.

18. Sewage treatment plant, exhibiting an irradiation device according to one of Claims 1 to 17.


**Revendications**

1. Dispositif pour exposer à des rayonnements ultraviolets un liquide absorbant dans un écoulement, présentant au moins deux sous-groupes de génération de rayons ultraviolets ayant chacun une source de rayonnements ultraviolets (4) cylindrique autour de laquelle sont disposés trois tubes de gainage (1, 2, 3) concentriques, un agent réfrigérant s'écoulant entre un tube de gainage intérieur (3) et un tube de gainage médian (2) parmi les tubes de gainage pour dévier la chaleur de la source de rayonnements ultraviolets, le liquide à exposer au rayonnement s'écoulant autour du tube de gainage extérieur (1) parmi les trois tubes de gainage et n'entrant en contact qu'avec celui-ci.

2. Dispositif selon la revendication 1, dans lequel un remplissage de gaz est prévu dans la cavité entre le tube de gainage extérieur (1) et le tube de gainage médian (2).

3. Dispositif selon la revendication 1 ou 2, dans lequel le diamètre extérieur du tube de gainage extérieur (1) est compris entre 80 et 250 mm.

4. Dispositif selon une des revendications 1 à 3, dans lequel la source de rayonnements ultraviolets (4) présente une densité de puissance d'au moins 10 watt de rayonnements ultraviolets C par cm dans la direction axiale.

5. Dispositif selon l'une des revendications 1 à 4, dans lequel la source de rayonnements ultraviolets est un générateur de rayons à moyenne pression de mercure.

6. Dispositif selon l'une des revendications 1 à 5, dans lequel les sources de rayonnements ultraviolets cylindriques, au moins au nombre de deux, sont disposées à l'horizontale transversalement au sens d'écoulement du liquide absorbant.

7. Dispositif selon la revendication 6, dans lequel les sources de rayonnements ultraviolets (4) cylindriques, au moins au nombre de deux, sont mises en place des deux côtés sur une paroi en acier (12) en forme de U munie de perforations (18) circulaires.

8. Dispositif selon la revendication 7, dans lequel les deux extrémités des trois tubes de gainage (1, 2, 3) concentriques sont supportées de façon à être étanches vis-à-vis de la paroi en acier (12) en forme de U, des raccordements pour l'énergie électrique et l'agent réfrigérant étant situés au niveau de ces extrémités.

9. Dispositif selon l'une des revendications 1 à 8, présentant des couvercles (8, 9, 10) qui empêchent une sortie vers le haut des rayonnements ultraviolets.

10. Dispositif selon la revendication 8, présentant au moins une fosse de montage (16, 17) disposée latéralement dans le sens de l'écoulement du liquide absorbant, fosse de montage qui permet la maintenance et le remplacement des sources de rayonnements ultraviolets (4) sans le démontage de pièces en contact avec le liquide.

11. Dispositif selon la revendication 6, dans lequel plusieurs sources de rayonnements ultraviolets sont disposées dans la coupe longitudinale selon l'échantillon d'une garniture cylindrique bidimensionnelle extrêmement étanche.

12. Dispositif selon l'une des revendications 1 à 11, présentant une pompe de recirculation de réfrigérant (22) ainsi qu'un échangeur de chaleur (23) pour fournir, au courant du liquide à absorber, l'énergie thermique prélevée sur le réfrigérant.

13. Dispositif selon la revendication 12, dans lequel l'échangeur de chaleur (23) sert simultanément de blindage contre les rayonnements ultraviolets sortant vers l'avant dans le sens de l'écoulement, et d'obstacle à l'écoulement afin d'homogénéiser le flux de la zone de rayonnement.

14. Dispositif selon l'une des revendications 1 à 13, dans lequel de l'eau distillée additionnée d'alcool est utilisée comme agent réfrigérant.

15. Dispositif selon l'une des revendications 1 à 14, présentant une régulation, dépendant du débit, de la puissance des sources de rayonnements ultraviolets (4).

16. Dispositif selon l'une des revendications 1 à 15, dans lequel le passage libre entre les tubes de gainage extérieurs (1) de deux sources de rayonnements ultraviolets (4) disposées l'une sur l'autre est choisi de sorte que la vitesse tangentielle du liquide sur la surface du tube de gainage extérieur, exprimée en m/s, est plus grande que la valeur numérique de la densité de puissance des rayonnements ultraviolets sortant à travers le tube de gainage extérieur (1), exprimée en $W/cm^2$.

17. Dispositif selon l'une des revendications 1 à 16, dans lequel le liquide absorbant est constitué d'eaux usées à désinfecter.

18. Installation d'épuration des eaux usées, présentant un dispositif d'exposition aux rayonnements selon l'une des revendications 1 à 17.

Fig. 1

EP 1 817 063 B1

Fig. 2

Fig. 3

Fig. 4

Fig. 5

**Fig. 6**

4

1

Punkt 2: 117 mW/cm2

Punkt 1: 160 mW/cm2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5124131 A
- US 5505912 A